# EUROPÄISCHE PATENTSCHRIFT

(11) **EP 1 414 407 B1**
(45) Veröffentlichungstag und Bekanntmachung des Hinweises auf die Patenterteilung: **20.02.2008**
(21) Anmeldenummer: 02764668.6
(22) Anmeldetag: 11.07.2002
(51) Int. Cl.: A61K 9/70, A61K 9/14, A61K 9/16, A61K 9/50

(54) **TRÄGERMATRIX MIT WIRKSTOFFBELADENEN PARTIKELN ZUR ANWENDUNG AUF DER HAUT ODER SCHLEIMHAUT**
DOSAGE FORMS COMPRISING PARTICLES CONTAINING ACTIVE INGREDIENT FOR APPLICATION ON THE SKIN OR MUCOUS MEMBRANE
FORMES POSOLOGIQUES RENFERMANT DES PARTICULES CONTENANT UN PRINCIPE ACTIF, DESTINEES A ETRE UTILISEES SUR LA PEAU OU LES MUQUEUSES

(30) Priorität: 27.07.2001 DE 10136784
(43) Veröffentlichungstag der Anmeldung: 06.05.2004
(73) Patentinhaber: LTS LOHMANN Therapie-Systeme AG, 56626 Andernach (DE)
(72) Erfinder: BECHER, Frank, 56072 Koblenz (DE)
(74) Vertreter: Flaccus, Rolf-Dieter
(86) Internationale Anmeldenummer: PCT/EP2002/007716
(87) Internationale Veröffentlichungsnummer: WO 2003/011247

(56) Entgegenhaltungen:
- WO-A-94/07468
- US-A- 5 071 645
- US-A- 5 230 898
- US-A- 5 456 917

## Beschreibung

Die Erfindung bezieht sich auf Darreichungsformen zur Anwendung auf der Haut oder Schleimhaut, mit einer Trägermatrix und mindestens einem in Partikeln vorliegenden Wirkstoff. Sie bezieht sich insbesondere auf Darreichungsformen zur transdermalen, transmucosalen oder epikutanen (topischen) Applikation von Wirkstoffen, wie transdermale therapeutisches Systeme (TTS), topische Wirkstoffpflaster oder transmucosale Therapiesysteme.

Bei der Herstellung von Darreichungsformen der vorstehend genannten Art wird vielfach so vorgegangen, daß der Wirkstoff in flüssiger Form, mit geeigneten Trägerstoffen und Hilfsstoffen vermischt, in das Grund- oder Matrixmaterial der Arzneiform eingearbeitet wird und sodann durch weitere Verfahrensschritte die gewünschte Darreichungsform hergestellt wird.
Diese Vorgehensweise ist aber aus verschiedenen Gründen nachteilig, insbesondere bei der Herstellung bestimmter Arten von Darreichungsformen.

Beispielsweise handelt es sich bei TTS, Wirkstoffpflastern oder transmucosalen therapeutischen Systemen im allgemeinen um flache Darreichungsformen von geringer Dicke, die zudem bestimmte physikalische Eigenschaften aufweisen müssen, wie Festigkeit, Elastizität, Klebrigkeit oder mucoadhäsive Eigenschaften.
Wird bei der Herstellung solcher Arzneiformen ein flüssiger Wirkstoff oder eine Wirkstofflösung in die Trägermatrix eingearbeitet, so kann dies zur Folge haben, daß im Falle einer zu hohen Wirkstoffbeladung die mechanischen Eigenschaften des Trägermaterials, insbesondere die Kohärenz und Flexibilität, wie auch die haftklebenden Eigenschaften beeinträchtigt werden.
Aufgrund der geringen Dicke dieser Systeme können deshalb im allgemeinen nur relativ geringe Mengen an flüssigem Wirkstoff eingearbeitet werden können.

Ferner ist dabei zu bedenken, daß bestimmte Wirkstoffe bei den für die Arzneimittelherstellung geeigneten Temperaturen ausschließlich als Flüssigkeiten vorliegen, so daß sie nur in dieser Form verarbeitet werden können.

WO-A-9407468 beschreibt transdermale Pflaster zur Wirkstoffabgabe, die eine Zwei-Phasen-Matrix aufweisen. Eine der beiden Phasen ist eine hydrophobe Polymerphase; die andere Phase besteht aus hydratisierten anorganischen Silikat-Partikeln, die in der Polymerphase dispergiert sind. In der absorbierten wässrigen Phase der hydratisierten Silikat-Partikel ist ein hydrophiler, wasserlöslicher Wirkstoff enthalten, der zumindest teilweise in der wässrigen Phase gelöst ist.

US-A-5071645 offenbart eine Wirkstoffabgabe-Vorrichtung, die ein mikroporöses Material umfaßt, das eine im wesentlichen aus UHMW-Polyolefin bestehende Matrix aufweist. In dieser sind feinpartikuläre Füllstoffe enthalten; der Anteil von Silica-Partikeln beträgt mindestens 50 Gew.-%. Außerdem ist die Matrix mit Poren versehen, die miteinander in Verbindung stehen. Ferner enthält die Vorrichtung mindestens einen Wirkstoff, der wenigstens mit einem Teil der Füllstoffpartikel verbunden ist.

US-A-5456917 bezieht sich auf implantierbare Biomaterialien zur verzögerten Freigabe von darin enthaltenen Wirkstoffen. Die Herstellung erfolgt in der Weise, daß ein spezielle, biologisch abbaubares Polymermaterial zu feinen Partikeln vermahlen wird und diese Partikel bei erhöhten Temperaturen extrudiert werden, um die gewünschte Form zu erhalten. Das Beladen der Partikel mit Wirkstoffen kann in der Weise erfolgen, daß die Polymerpartikel in einer wirkstoffhaltigen Lösung dispergiert werden, woraufhin ein Vakuum angewandt wird, um die Hohlräume der Partikel mit der wirkstoffhaltigen Lösung zu füllen.

US-A-5230898 offenbart ein transdermales therapeutisches System, das eine Matrix aus wasser-unlöslichem Material aufweist, worin Inseln oder Einschlüsse verteilt sind, die im wesentlichen aus einer festen Lösung eines Arzneistoffs in einem wasserlöslichen oder in Wasser quellfähigen Grundmaterial bestehen. Als Grundmaterial kommen beispielsweise Polyvinylalkohol, Polyvinylpyrrolidon, Copolymere der Polymethacrylsäure, Polysaccharide, Polyethylenglykol, und homogene Mischungen dieser Stoffe in Betracht.

Der vorliegenden Erfindung lag deshalb die Aufgabe zugrunde, Darreichungsformen oder Arzneizubereitungen der eingangs genannten Art aufzuzeigen, bei deren Herstellung von flüssigen Wirkstoffzubereitungen ausgegangen werden kann, ohne daß jedoch die vorstehend genannten Nachteile in Erscheinung treten.

Diese Aufgabe wird durch Arzneizubereitungen gemäß Anspruch 1 und die Herstellungsverfahren nach den Ansprüchen 18 bis 22 gelöst, sowie durch die in den abhängigen Ansprüchen beschriebenen, besonders bevorzugten Ausführungsformen.

Die Erfindung sieht vor, daß die Trägermatrix einer im Oberbegriff des Anspruchs 1 genannten Darreichungsform eine Vielzahl von offen-porösen oder Kapillarräume enthaltenden Partikeln aufweist, wobei diese Partikel als Wirkstoffreservoir dienen.

Ein besonderer Vorteil dieser Art von Darreichungsformen liegt darin, daß der jeweilige Wirkstoff nicht gleichmäßig in der Trägermatrix verteilt oder gelöst aufgebracht werden muß, sondern sich in vielen kleinen Reservoirpartikeln befindet. Dadurch kann die Gesamtmenge des Wirkstoffes erheblich reduziert werden, da es nicht notwendig ist, ihn homogen zu verteilen. Es genügt, wenn er in vielen kleinen Partikeln, und zwar nur in diesen, in hinreichender Konzentration vorliegt, um seine Wirksamkeit zu entfalten.
Ein weiterer Vorteil ist, daß die Festigkeit der Darreichungsform bzw. der Trägermatrix durch den in Partikelform vorliegenden Wirkstoff nicht beeinträchtigt wird, da die flüssigen Wirkstoff-Anteile in den Partikeln gebunden sind.
1. Die erfindungsgemäßen Partikel können offen-poröse oder Kapillarräume enthaltende Partikel mit großer innerer Oberfläche sein.
2. Sie können auch solche wirkstoffhaltigen Partikel sein, die durch das in WO 99/17868 beschriebene Verfahren gewonnen werden.

Nachstehend wird zunächst auf die erstgenannte Form der Partikel eingegangen.

Diese Partikel dienen als Wirkstoffreservoir und enthalten mindestens einen Wirkstoff, vorzugsweise in flüssiger Form. Unter "flüssiger Form" wird verstanden, daß der Wirkstoff selbst im flüssigen Aggregatszustand vorliegt, oder daß er als Lösung, Dispersion, Suspension, Emulsion oder als flüssige Wirkstoffzubereitung vorliegt.

Ein Hauptvorteil dieser Arzneizubereitungen besteht darin, daß die wirkstoffhaltigen Partikel zunächst mit flüssigem Wirkstoff oder einer flüssigen Wirkstoffzubereitung in einer dem Fachmann bekannten Form beladen werden.

Dies kann insbesondere in einer besonders bevorzugten Ausführungsform dadurch geschehen, daß die porösen oder Kapillarräume aufweisenden Partikel in ein Vakuum (vorzugsweise im Bereich von ca. 100 bis 10-3 mbar, stärker bevorzugt 10 bis 0,01 mbar, am meisten bevorzugt 1 bis 0,1 mbar) verbracht werden. Dies hat den besonderen Vorteil, daß Luft, die sich meist in den Kapillaren befindet, entfernt wird; dadurch ist das spezifische Gewicht der Partikel größer und sie schwimmen nicht mehr auf der Oberfläche der Wirkstoffflüssigkeit. Noch im Vakuum werden die Partikel mit der Wirkstoffflüssigkeit quasi umspült, was z.B. durch Rühren mit Hochgeschwindigkeitsrührern, Schütteln oder in sonstiger geeigneter Weise erreicht werden kann. Werden danach normale Druckverhältnisse hergestellt, so wird die Wirkstoffflüssigkeit vom Luftdruck in die Kapillaren oder Poren hineingepreßt.

In einer weiteren bevorzugten Ausführungsform werden die Partikel in die Wirkstoffflüssigkeit eingebracht, und diese wird dann unter erhöhten Druck (vorzugsweise im Bereich von 2 bis 300 bar, stärker bevorzugt 10 bis 200 bar, am meisten bevorzugt 10 bis 100 bar) gesetzt, so daß die Wirkstoffflüssigkeit in die luftgefüllten Poren hineingepreßt wird. Bei der anschließenden Entspannung dringt die in den Poren befindliche Luft heraus, weil die Adhäsionskräfte der Flüssigkeit größer sind.

Man kann die dem Fachmann bekannten Verfahren zur Imprägnierung einsetzen (wie die Kesseldruckimprägnierung von Holz).

In einer weiteren bevorzugten Ausführungsform werden die Partikel auf hohe Temperaturen (vorzugsweise im Bereich von 40 bis 200 °C, besonders bevorzugt 50 bis 150 °C) gebracht, so daß der Druck der in den Poren befindlichen Luft gering ist; diese heißen Partikel werden mit kalter Wirkstoffflüssigkeit umspült, so daß sie in die Hohlräume eindringen kann. "Kalt" bedeutet, daß die Temperatur niedriger ist als diejenige der Partikel.

Ferner kann die Beladung der Partikel in der Weise geschehen, daß die Partikel unter Normaldruck und bei Raumtemperatur (ca. 20-30 °C) in dem flüssigen Wirkstoff bzw. der flüssigen Wirkstoffzubereitung suspendiert und vermischt werden, vorzugsweise unter Rühren.

Die oben angegebenen Verfahren lassen sich in dem Fachmann bekannter Weise kombinieren, beispielsweise durch abwechselnde Druck- und Vakuum-Imprägnierung.
Die beladenen Partikel werden, soweit erforderlich, von der überschüssigen Wirkstoffflüssigkeit abgetrennt, beispielsweise durch Sedimentation oder Filtration.

Anschließend können die mit flüssigem Wirkstoff beladenen Partikel in fester Form, z.B. als Pulver, in die Trägermatrix der jeweiligen Arzneizubereitung eingearbeitet werden. Dabei wird außer der in den Partikeln eingeschlossenen Wirkstoffflüssigkeit keine Flüssigkeit, oder nur unwesentliche Mengen, in die Trägermatrix-Masse eingetragen, so daß die Struktur, Konsistenz, Klebrigkeit, Elastizität und sonstige Eigenschaften des Matrixmaterials nicht nachteilig beeinflußt werden. Jedenfalls ist es auf diese Weise möglich, größere Mengen eines in flüssiger Form vorliegenden Wirkstoffes in eine Arzneizubereitung einzuarbeiten, als dies bei konventioneller Herstellungsweise der Fall wäre. Bei der Herstellung der erfindungsgemäßen Arzneiformen können deshalb im wesentlichen dieselben Methoden und Apparaturen verwendet werden, die für die Verarbeitung fester Arzneimittelwirkstoffe eingesetzt werden.
Insbesondere wird durch die Erfindung die Herstellung von flachen, dünnen Darreichungsformen wie transdermalen therapeutischen Systemen oder mucoadhäsiven Arzneiformen, ausgehend von flüssigen Wirkstoffzubereitungen, ermöglicht.

Besonders vorteilhaft ist es, daß die verschiedenen Partikel auch mit verschiedenen Wirkstoffen beladen werden können, so daß in einfacher Weise Kombinationspräparate hergestellt werden können, wie z.B. Gestagen-Östrogen-Pflaster.

Besonders vorteilhaft ist es auch, daß man andere Partikel auch zusätzlich mit flüssigen Weichmachern und/oder (Hautpenetrations-)Enhancern beladen kann. Alternativ können diese Substanzen aber auch gemeinsam mit dem Wirkstoff in denselben Partikeln enthalten sein.
Weichmacher bzw. Enhancer können aus den folgenden Stoffen bzw. Stoffgruppen ausgewählt werden:
Gesättigte oder ungesättigte Fettsäuren, Kohlenwasserstoffe, geradkettige oder verzweigtkettige Fettalkohole, Dimethylsulfoxid, Propylenglykol, Decanol, Dodecanol, 2-Octyldodecanol, Glycerin, Isopropylidenglycerol, Transcutol (= Diethylenglycol-monoethylether), DEET (= N,N-Diethyl-m-Toluolamid), Solketal, Ethanol, 1,2-Propandiol oder andere Alkohole, Menthol und andere ätherische Öle oder Bestandteile ätherischer Öle, Laurinsäurediethanolamid, D-alpha-Tocopherol und Dexpanthenol; die vorstehende Aufzählung ist nicht abschließend.

Man kann vorteilhaft verschiedene Partikel-Typen und -Größen einsetzen, um ein differenziertes Freisetzungsverhalten zu erreichen.

Ein weiterer Vorteil ist, daß durch die Verwendung von flüssigkeitsgefüllten Wirkstoffpartikeln die Sicherheit bei der Produktion von Arzneimitteln verbessert werden kann. Dies gilt insbesondere dann, wenn für das Personal das Risiko einer Kontamination mit Wirksubstanzen, insbesondere mit toxischen Substanzen, besteht.

Als poröse Partikel, welche mit flüssigen Wirkstoffen oder Wirkstofflösungen beladen werden können, eignen sich vor allem solche, die aus der Gruppe ausgewählt sind, die Aktivkohle-Partikel, Partikel aus porösen Mineralien, insbesondere Kieselgur-Partikel, Diatomeenerde, Bims, Lava, Bentonit, Keramik- oder Tonpartikel, Kieselgel-Partikel, Silicium-Monoxid-Partikel, Zeolithe sowie Partikel aus natürlichen oder synthetischen Schwämmen oder aus verfestigten Schäumen umfaßt. Unter porösen Partikeln werden auch solche verstanden, die eine Kapillarstruktur aufweisen.
Eine gemeinsame Eigenschaft der genannten Partikel ist, daß sie durch die vorhandenen Poren oder Kapillaren eine große innere Oberfläche aufweisen, welches eine Grundvoraussetzung für eine hohe Wirkstoffbeladung ist.

Als synthetische Schwämme oder Schäume kommen, abhängig von der beabsichtigten Anwendung, sowohl biodegradierbare Materialien (z.B. verfestigte Gelatine- oder Kollagen-Schäume) als auch nicht degradierbare Materialien (z.B. polyurethanSchäume, mikrozellulare Polyester- oder Polyetherschäume) in Betracht.
Ferner kommen auch Superabsorber, wie quellbare Polymere, in Betracht, wie sie beispielsweise in PCT/EP 95/02120 beschrieben sind.

Bei der Auswahl der Partikel ist darauf zu achten, daß der gewählte Typ für die beabsichtigte Applikationsart (z.B. oral, transmucosal) unter pharmakologischen und toxikologischen Gesichtspunkten geeignet und unbedenklich ist. Dabei sind auch mögliche Wechselwirkungen mit dem verwendeten Wirkstoff, welche dem Fachmann bekannt sind, zu berücksichtigen und möglichst zu vermeiden.
Die durchschnittliche Teilchengröße der porösen Partikel ist vorzugsweise ≤ 2 mm, stärker bevorzugt ≤ 0,5 mm, noch stärker bevorzugt ≤ 200 µm, insbesondere ≤ 50 µm.
Die Partikelgröße kann z.B. durch Mahlen und/oder Sieben, aber auch durch Züchten geeigneter Kristalle oder durch dem Fachmann bekannte geeignete Fällungsverfahren eingestellt werden.

Die erfindungsgemäß verwendeten Partikel sind im allgemeinen feinporig, wobei der durchschnittliche Poren- oder Kapillarendurchmesser bevorzugt ≤ 0,1 mm, stärker bevorzugt ≤ 20 µm und besonders bevorzugt ≤ 1 µm ist.

Der Anteil der wirkstoffbeladenen Partikel, bezogen auf die Trägermatrix, kann in einem weiten Bereich variiert werden. Um eine hohe Wirkstoffbeladung zu erreichen, können die wirkstoffbeladenen Partikel in einem Anteil von bis zu 95 Gew.-% in einer Darreichungsform enthalten sein, abhängig von der jeweils ausgewählten Trägermatrix. Der Partikelanteil beträgt deshalb bevorzugt 0,1 bis 95 Gew.-%, stärker bevorzugt 5 bis 60 Gew.-%, besonders bevorzugt 5 bis 25 Gew.-%, jeweils bezogen auf die gesamte Darreichungsform. Durch die große Spannbreite hinsichtlich des Partikel-Gehalts, wie auch bezüglich der Wirkstoffmenge bzw. -konzentration in den einzelnen Partikeln können die erfindungsgemäßen Darreichungsformen einen breiten Bandbereich hinsichtlich der Dosierung abdecken.
Allerdings ist dabei zu berücksichtigen, daß ein übermäßig hoher Anteil an wirkstoffbeladenen Partikeln sich nachteilig auf die physikalischen Eigenschaften der Trägermatrix auswirken kann. Die Obergrenze für diesen Anteil läßt sich im Einzelfall leicht experimentell ermitteln.

Die Beladung dieser porösen oder Kapillaren aufweisenden Partikel mit Wirkstoff(en) kann bevorzugt auf die Weise erfolgen, daß der flüssige Wirkstoff, eine Wirkstofflösung, -dispersion, -suspension oder -emulsion, oder eine flüssige Wirkstoffzubereitung, mit einer geeigneten Menge von Partikeln vermischt wird, wodurch die Poren- oder Kapillarräume mit Wirkstoffflüssigkeit oder -lösung gefüllt werden. Anschließend können die beladenen Partikel durch dem Fachmann bekannte Methoden von der überschüssigen Wirkstoffflüssigkeit bzw. -lösung abgetrennt werden. Wahlweise kann sich daran ein Trockenvorgang anschließen, um noch vorhandene Flüssigkeits- oder Lösemittelreste zu entfernen.

Nach einer bevorzugten Ausführungsform wird eine wirkstoffhaltige Lösung verwendet, welche mindestens einen festen Wirkstoff in gelöster Form in einem geeigneten Lösemittel enthält. Hierbei kann es sich auch um eine gesättigte Wirkstofflösung handeln.
Um bei der Beladung der Partikel das Eindringen der Wirkstoffflüssigkeit in das Innere der Partikel zu erleichtern, kann es notwendig sein, geringe Mengen von Tensiden oder Emulgatoren hinzuzufügen.

Neben den bereits genannten porösen Partikeln werden für die Herstellung der erfindungsgemäßen Darreichungsformen insbesondere auch solche wirkstoffhaltige Partikel bevorzugt, die durch das in WO 99/17868 beschriebene Verfahren erhalten werden können und als "Concentrated Powder Form" (CPF)-Partikel bezeichnet werden. Dabei handelt es sich um pulverförmige, flüssigkeitsbeladene Partikel oder Agglomerate von Partikeln, welche entstehen, wenn ein inertes Gas oder Gasgemisch in einem flüssigen Wirkstoff, einer wirkstoffhaltigen Lösung oder Suspension oder sonstigen flüssigen Wirkstoffzubereitung unter Druck (vorzugsweise ca. 5 bis 500 bar, besonders bevorzugt im Bereich von 10 bis 250 bar) in dieser Flüssigkeit gelöst wird, und diese Lösung anschließend schnell entspannt wird (beispielsweise durch eine Düse), wobei gleichzeitig ein pulverförmiges festes Trägermaterial (Trägerpartikel) beigemischt wird. Die so erhaltenen Pulver sind im wesentlichen trocken und rieselfähig und können bis zu 80 Gew.-% einer Wirkstoffflüssigkeit enthalten. Sie haben den Vorteil, daß sie trotz eines hohen Flüssigkeitsgehaltes wie Feststoffpartikel verarbeitet werden können.
Die enthaltene Flüssigkeit befindet sich entweder in den Kapillarräumen der aggregierten Trägerpartikel, und/oder in den Poren der Trägerpartikel, falls offenporige Trägerpartikel verwendet werden.

Als pulverförmige Trägerstoffe bzw. -partikel können beispielsweise Stärke-Arten (wie Mais-, Kartoffel-, Weizenstärke), Kieselsäure bzw. Siliciumdioxid, Cellulosen (z.B. mikrokristalline Cellulosen, Cellulosederivate wie Carboxymethylcellulose, Cellulosefasern) verwendet werden; darüber hinaus können als Trägerpartikel auch poröse Partikel der eingangs erwähnten Art verwendet werden, wie z.B. Aktivkohle, Zeolithe, Kieselsäure, oder quellbare Polymere (insbesondere sogenannte Superabsorber-Polymere). Als quellbare Polymere werden vorzugsweise wasserquellbare Polymere verstanden, z.B. Polyvinylalkohol mit hohem Hydrolysegrad oder hochmolekulare Hydroxypropylmethylcellulose.
Die pulverförmigen Trägerpartikel weisen vorzugsweise eine Partikelgröße von weniger als 100 µm auf.

Als weitere Hilfsstoffe bei der Herstellung der pulverförmigen flüssigkeitsgefüllten Partikel kommen in Betracht: Kochsalz, Zucker, Dextrin, Proteine, Titandioxid, Fette, Polyglykole, Magnesiumstearat, hochdisperses Siliciumdioxid, Glutamat, Kalk, Kaolin, Polymilchsäure, Fette, Wachse, Verdickungsmittel.

Um ein nachfolgendes Resuspendieren der flüssigkeitsgefüllten Partikel zu erleichtern, können bei der Herstellung auch Emulgatoren wie z.B. Phospholipide, insbesondere Lecithin, oder Partialglyceride zugesetzt werden.

Als inerte Gase kommen vor allem Kohlendioxid, gasförmige Kohlenwasserstoffe (z.B. Methan, Ethan, Propan, Butan), Ether, Stickstoff, Distickstoffoxid, Ammoniak oder Edelgase in Betracht.
Auch die nach dem beschriebenen Verfahren beladenen Partikel werden, soweit erforderlich, von der überschüssigen Wirkstoffflüssigkeit abgetrennt, beispielsweise durch Sedimentation oder Filtration.

Die weitere Verarbeitung und Verwendung dieser Partikel, insbesondere die Herstellung der erfindungsgemäßen Darreichungsformen, kann auf entsprechende Weise erfolgen wie weiter oben für die erstgenannte Form der Partikel beschrieben.

Die nachfolgenden Ausführungen gelten für alle verschiedenen beschriebenen Typen von Partikeln:

Gemäß einer bevorzugten Ausführungsform ist des weiteren vorgesehen, daß die Partikel, oder zumindest eine Teilmenge davon, nach der Wirkstoffbeladung mit einem Überzug aus fett- und/oder wasserlöslichen Stoffen versehen werden. Auf diese Weise kann beispielsweise eine Steuerung der Wirkstofffreisetzung, insbesondere eine Steuerung der Freisetzungsgeschwindigkeit, erreicht werden, oder es kann die Wasserbenetzbarkeit verbessert werden. Als Materialien für solche Überzüge kommen u.a. in Betracht: Filmbildner (z.B. Polyacrylate, Polymethacrylate), Polyethylenglykole, pflanzliche oder tierische Öle, flüssiges Paraffin, Polyvinylpyrrolidon, Cellulosederivate.

Um die Wirkstofffreisetzung bei der Applikation zu beschleunigen, kann es vorteilhaft sein, wenn als Material für die genannten Partikel ein wasserlösliches oder biodegradierbares Material gewählt wird. Dann kann durch Einwirkung von Körperflüssigkeiten (z.B. Schweiß, Speichel, Schleim) oder Enzymen ein Abbau der Struktur der Wirkstoffpartikel stattfinden, wodurch der enthaltene Wirkstoff schneller freigesetzt wird.

Die vorliegende Erfindung bezieht sich insbesondere auf Darreichungsformen zur Anwendung auf der Haut, welche als transdermale therapeutische Systeme (TTS) formuliert sind. Diese sind im allgemeinen flächenförmig aufgebaut und ermöglichen die Verabreichung von systemisch wirkenden Arzneimittelwirkstoffen über die Haut, wobei die Wirkstoffe über einen festgelegten Zeitraum mit einer definierten Freisetzungsrate kontinuierlich an die Haut abgegeben werden können.

Die erfindungsgemäßen TTS umfassen eine wirkstoffundurchlässige Rückschicht und eine damit verbundene Trägermatrix, wobei in die Trägermatrix eine Vielzahl von offen-porösen oder Kapillarräume enthaltenden Partikeln eingebettet sind, die als Wirkstoffreservoir dienen und mindestens einen Wirkstoff in flüssiger Form enthalten, wie oben beschrieben.

Als Grundmaterialien für die Herstellung der Trägermatrix, in welche die Partikel eingebettet werden, können im allgemeinen alle diejenigen Polymermaterialien verwendet werden, die nach dem Stand der Technik zur Herstellung von Wirkstoffreservoir-Schichten von TTS verwendet werden. Insbesondere können folgende Stoffe für die Herstellung der Trägermatrix verwendet werden: Polyacrylate, Poly(meth)acrylate, Copolymere aus Acryl- und Methacrylderivaten und Vinylverbindungen (z.B. unter Verwendung folgender Monomere: Acrylsäure, Methacrylsäure, Acrylsäureethylester, Acrylsäurebutylester, Acrylsäureoctylester, 2-Ethyl-hexylacrylat, 2-Hydroxyethylacrylat und Vinylacetat); ferner Polysiloxane, bevorzugt selbstklebende Polysiloxane, Silikonkautschuke; Kohlenwasserstoffpolymere, vorzugsweise Polyisobutylen, Polyisopren, Styrol-Isopren-Styrol-Blockcopolymere und StyrolButadien-Styrol-Blockcopolymere; haftklebende Zubereitungen auf der Basis von Cellulosederivaten (z.B. Ethylcellulose, Hydroxypropylcellulose, Hydroxypropylmethylcellulose) und Klebharzen (z.B. Kolophonium und -derivate). Unter Verwendung der vorstehend genannten Materialien können Trägermatrices mit haftklebenden Eigenschaften hergestellt werden; solche auf der Haut haftklebenden Formulierungen sind dem Fachmann bekannt. Die haftklebende Oberfläche des TTS, mittels welcher das TTS auf der Haut befestigt wird, ist im Zustand vor der Applikation mit einer ablösbaren Schutzfolie bedeckt.

Als Hilfsstoffe können der Trägermatrix beispielsweise Weichmacher, Füllstoffe und hautpenetrationsfördernde Substanzen (Penetrations-Enhancer) beigemischt werden. Hierfür geeignete Substanzen sind dem Fachmann bekannt.

Um die erfindungsgemäßen TTS herzustellen, können die Trägermatrix-Materialien sowohl in gelöster Form als auch als Schmelze verarbeitet werden, sofern es sich um Schmelzkleber handelt. In beiden Fällen werden die mit Wirkstoffflüssigkeit gefüllten porösen Partikel homogen in die noch flüssige oder halbfeste Trägermatrix-Zubereitung eingearbeitet. Falls erforderlich, können Netzmittel (Tenside, z.B. SDS), Emulgatoren (z.B. Lecithin) etc. beigemischt werden, um die Dispergierung der Partikel in dem Trägermatrix-Material zu verbessern.

Als Material für die Rückschicht eignen sich eine Vielzahl von hautverträglichen Kunststoffolien, wie z.B. Folien aus Polyvinylchlorid, Ethylenvinylacetat, Vinylacetat, Polyethylen, Polypropylen oder Cellulosederivaten. Besonders geeignet als Material für die Rückschicht sind Polyesterfolien (z.B. Polyethylenterephthalat). Die genannten Folien eignen sich auch als Material für die ablösbare Schutzfolie, vorausgesetzt, daß sie durch geeignete Oberflächenbehandlung, wie z.B. Silikonisierung, ablösbar gemacht sind.

Gemäß einer weiteren Ausführungsform ist vorgesehen, daß die Trägermatrix einen zwei- oder mehrschichtigen Aufbau aufweist, wobei in mindestens einer Schicht eine Vielzahl von offen-porösen oder Kapillarräume enthaltenden Partikeln eingebettet sind, die als Wirkstoffreservoir dienen und mindestens einen Wirkstoff in flüssiger Form enthalten.

Die erfindungsgemäß vorgeschlagenen flüssigkeitsgefüllten porösen Partikel lassen sich in entsprechender Weise auch zur Herstellung von TTS verwenden, die ein beutelförmiges Wirkstoffreservoir aufweisen, welches mit einer flüssigen, hochviskosen, halbfesten oder thixotropen wirkstoffhaltigen Matrix gefüllt ist, beispielsweise einem Gel. In diesem Fall werden die flüssigkeitsgefüllten Partikel in die Matrix des beutelförmigen Wirkstoffreservoirs eingebettet.

Weitere besonders bevorzugte Ausführungsformen der Erfindung betreffen mucoadhäsive Darreichungsformen, z.B. mucoadhäsive Pflaster bzw. Systeme. Diese können zwecks Wirkstoffverabreichung auf Schleimhautoberflächen (z.B. Mund-, Nasen-, Vaginalschleimhaut) appliziert werden, auf denen sie haften bleiben. Sie weisen eine fest oder halbfeste schleimhauthaftende Trägermatrix auf, in der die wirkstoffhaltigen Partikel eingebettet oder dispergiert sind. Sie sind im wesentlichen flach und können, ebenso wie TTS, auf der Rückseite (der schleimhauthaftenden Seite gegenüberliegend) mit einer Rückschicht aus einer Kunststofffolie versehen sein.

Für die Herstellung der mucoadhäsiven Trägermatrix kommen vorzugsweise folgende Materialien in Betracht: Cellulosederivate wie Carboxymethylcellulose-Natrium, Carboxymethylcellulose, Hydroxypropylcellulose, Methylcellulose, Ethyl- oder Propylcellulose; Polyvinylalkohol, Polyacrylsäure, Polyacrylate, Polyvinylpyrrolidone, Polyethylenoxid-Polymere; wasserlösliche Polysaccharide, die pflanzlichen oder mikrobiellen Ursprungs sind, insbesondere Pullulan, Xanthan, Alginate, Stärke, Dextrane und Pektine; Gelatine und andere gelbildende Proteine. Dem Fachmann sind geeignete mucoadhäsive Formulierungen, ausgehend von den genannten Stoffen, bekannt.

Weitere bevorzugte Ausführungsformen sind Wirkstoffpflaster zur topischen oder epikutanen Verabreichung von Wirkstoffen an die Haut. Diese können einen ähnlichen Aufbau aufweisen wie die beschriebenen TTS, mit einer festen oder halbfeste Trägermatrix, in die eine Vielzahl von offen-porösen oder Kapillarräume enthaltenden Partikeln eingebettet sind, die als Wirkstoffreservoir dienen und mindestens einen Wirkstoff enthalten, vorzugsweise in flüssiger Form.

In allen oben beschriebenen Fällen kann die Trägermatrix, in der die wirkstoffhaltigen Partikel eingebettet sind, neben den matrixbildenden Grundmaterialien wahlweise Hilfsstoffe enthalten. Hierfür kommen Füllstoffe (z.B. SiO₂); Verdikkungsmittel (z.B. Alginate, Pectin); Farbstoffe (z.B. Chinolingelb oder TiO₂); Emulgatoren (z.B. polyethoxylierte Sorbitanfettsäureester wie TWEEN^{®} oder polyethoxylierte Fettalkohole wie BRIJ^{®}); Hautpenetrations-Enhancer (siehe oben); Weichmacher (z.B. Polyethylenglykol, Glycerin); Süßstoffe (z.B. Aspartam, Saccharin); Konservierungsmittel (z.B. Sorbinsäure und deren Salze) und Aromastoffe in Betracht.

Unter Wirkstoffen werden alle im Bereich der Human- oder Tiermedizin eingesetzten Arzneistoffe verstanden, einschließlich Vitamine, Enzyme und Hormone, sowie Wirkstoffe für kosmetische Behandlungen und Geschmacks- oder Aromastoffe. Insbesondere bezieht sich die Erfindung auf Arzneimittelwirkstoffe, die über die Haut oder Schleimhaut aufgenommen werden können.
Besonders bevorzugt sind Wirkstoffe, die im flüssigen Zustand vorliegen; darüber hinaus ist die Erfindung auf eine Vielzahl weiterer Wirkstoffe anwendbar, die in eine flüssige Form gebracht werden können, beispielsweise als Lösung, Dispersion, Suspension oder Emulsion.
Die Abgabe des/der in den erfindungsgemäßen Arzneiformen enthaltenen Wirkstoffs/Wirkstoffe kann auf verschiedene Weise erfolgen. Bei transdermaler Verabreichung oder Applikation auf eine Schleimhautoberfläche kann der Wirkstoff aus den Partikeln herausdiffundieren und nachfolgend resorbiert werden. Falls die Darreichungsform zerfallsfähig oder degradierbar gestaltet ist, können die Partikel zunächst als solche freigesetzt werden, und nachfolgend der in den Partikeln enthaltene Wirkstoff freigesetzt werden. Falls die Partikel aus biodegradierbarem Material hergestellt sind, kann die Freisetzung durch Abbau des Partikelmaterials beeinflußt bzw. beschleunigt werden. Auf diese Weise werden durch die Erfindung vielerlei Möglichkeiten zur Steuerung der Wirkstoffabgabe eröffnet.
Des weiteren kann die Wirkstoffabgabe auch auf die Weise erfolgen, daß die Partikel aus der Trägermatrix der transdermalen oder transmucosalen Darreichungsform durch die Haut oder Schleimhaut migrieren oder diffundieren und nachfolgend den Wirkstoff in den Kreislauf abgeben.

Die Erfindung umfaßt ferner Verfahren zur Herstellung von Arzneiformen zur transdermalen, transmucosalen oder epikutanen Verabreichung, wobei von flüssigen Wirkstoffen, Wirkstofflösungen oder -zubereitungen ausgegangen wird.

Die erfindungsgemäßen Darreichungsformen können vorzugsweise auf die Weise erhalten werden, daß zunächst ein für die gewünschte Arzneiform geeignetes Trägermatrix-Material - wie oben beschrieben - bereitgestellt wird, vorzugsweise in flüssiger oder halbfester Form (z.B. als Lösung oder Schmelze), oder als Gel.
Des weiteren wird ein flüssiger Wirkstoff, eine Wirkstofflösung oder eine flüssige Wirkstoffzubereitung bereitgestellt. Falls der Wirkstoff nicht selbst flüssig vorliegt, wird dieser in einem pharmazeutisch akzeptablen und für den Wirkstoff geeigneten Lösemittel oder Lösemittelgemisch gelöst, dispergiert oder suspendiert. Die flüssigen Wirkstoffzubereitungen können ferner auch Wirkstoffkombinationen enthalten.

In einem nächsten Arbeitsschritt wird der flüssige Wirkstoffs bzw. die Wirkstofflösung mit offen-porösen oder Kapillarräume aufweisenden Partikeln (wie oben beschrieben) vermischt, wodurch die Poren- oder Kapillarräume mit Wirkstoffflüssigkeit oder -lösung gefüllt werden. Dieser Vorgang kann durch Zusatz von Tensiden oder Emulgatoren unterstützt werden.
Nach dem Abtrennen der Partikel von der überschüssigen Wirkstoffflüssigkeit bzw. -lösung, wahlweise gefolgt von einem Trocknungsschritt, werden die mit Wirkstoff-Flüssigkeit beladenen Partikel in das im ersten Schritt genannte Trägermaterial eingebracht und in dieses eingearbeitet und gemischt, so daß die Partikel homogen in der Trägermatrix verteilt sind. Falls erforderlich, können Netzmittel (Tenside, z.B. SDS), Emulgatoren (z.B. Lecithin) etc. beigemischt werden, um die Dispergierung der Partikel in dem Trägermatrix-Material zu verbessern.

Schließlich können, abhängig von der Art der herzustellenden Arzneiform, Hilfsstoffe (wie oben erwähnt) hinzugegeben und eingearbeitet werden, und es kann eine weitere Trocknung vorgenommen werden, um durch Lösemittelentzug die gewünschte Konsistenz der Trägermatrix herzustellen.
Die weitere Verarbeitung der Arzneiformen kann mittels konventioneller Methoden erfolgen, z.B. Pressen, Stanzen oder Beschichtung.

Das vorstehend beschriebene Verfahren kann auf verschiedene Weise abgewandelt werden. Beispielsweise können die mit Wirkstoff beladenen porösen Partikel vor der Einbettung in die Trägermatrix mit einem Überzug versehen werden, der eine Diffusion des Wirkstoffs in die Matrix (oder in das Lösemittel) verhindert, solange diese noch nicht getrocknet oder erstarrt ist. Ebenso können die Partikel, wie oben erwähnt, vor dem Einbetten mit einem fett- und/oder wasserlöslichem Überzug versehen werden.

In einem weiteren bevorzugten Herstellungsverfahren für Arzneiformen zur transdermalen, transmucosalen oder epikutanen Verabreichung ist in Abwandlung zu dem vorstehend beschriebenen Verfahren vorgesehen, daß die Herstellung der wirkstoffbeladenen Partikel nach dem in WO 99/17868 beschriebenen Verfahren erfolgt, wie oben beschrieben ("Concentrated Powder Form" (CPF)-Partikel).
Dieses wirkstoffhaltige Pulver wird sodann in das in flüssiger oder halbfester Form vorliegende Trägermaterial eingebettet; die weitere Verarbeitung erfolgt wie oben beschrieben. Weiterhin kann auch dieses Herstellungsverfahren auf verschiedene Weise abgewandelt werden, beispielsweise durch Aufbringen von Beschichtungen oder Überzügen auf die Partikel vor dem Einbetten.

Somit ermöglicht die vorliegende Erfindung in vorteilhafter Weise die Herstellung von transdermalen, epikutanen oder mucoadhäsiven Arzneiformen, insbesondere von flachen Arzneiformen, welche einen hohen Gehalt eines in flüssiger Form vorliegenden Wirkstoffs aufweisen können.

## Patentansprüche

1. Darreichungsformen zur Anwendung auf der Haut oder Schleimhaut, umfassend eine Trägermatrix mit haftklebenden oder mucoadhäsiven Eigenschaften, und mindestens einen Wirkstoff, wobei die Trägermatrix eine Vielzahl von Partikeln aufweist, welche als Wirkstoffreservoir dienen und mindestens einen Wirkstoff enthalten, **dadurch gekennzeichnet, daß** die genannten Partikel
- aus der Gruppe, die offen-poröse Partikel aus natürlichen Schwämmen, offen-poröse Partikel aus synthetischen Schwämmen sowie offen-poröse Partikel aus verfestigten Schäumen umfaßt, ausgewählt sind.

2. Darreichungsformen nach Anspruch 1, **dadurch gekennzeichnet, daß** die durchschnittliche Teilchengröße der Partikel ≤ 2 mm, vorzugsweise ≤ 0,5 mm, stärker bevorzugt ≤ 200 µm ist.

3. Darreichungsformen nach Anspruch 1 oder 2, **dadurch gekennzeichnet, daß** die porösen oder Kapillarräume aufweisenden Partikel feinporig sind, mit einem durchschnittlichen Poren- bzw. Kapillarendurchmesser von ≤ 0,1 mm, vorzugsweise von ≤ 20 µm insbesondere von ≤ 1 µm.

4. Darreichungsformen nach einem der vorangehenden Ansprüche, **dadurch gekennzeichnet, daß** der Anteil der Partikel, bezogen auf die Trägermatrix, 0,1 bis 95 Gew.-%, vorzugsweise 5 bis 60 Gew.-% beträgt.

5. Darreichungsform nach einem der vorangehenden Ansprüche, **dadurch gekennzeichnet, daß** die Partikel als Trägermaterial quellbare, flüssigkeitsabsorbierende Polymere, vorzugsweise superabsorbierende Polymere enthalten, wobei wasserquellbare Polymere, insbesondere Polyvinylalkohol mit hohem Hydrolysegrad und hochmolekulare Hydroxypropylmethylcellulose, besonders bevorzugt sind.

6. Darreichungsformen nach einem der vorangehenden Ansprüche, **dadurch gekennzeichnet, daß** das genannte pulverförmige Trägermaterial aus der Gruppe ausgewählt ist, die
- Stärke-Arten, insbesondere Mais-, Kartoffel-, Weizenstärke,
- Kieselsäure und Siliciumdioxid Cellulosen, insbesondere mikrokristalline Cellulosen, Cellulosederivate wie Carboxymethylcellulose, Cellulosefasern,
- Aktivkohle, Zeolithe und Kieselsäure
umfaßt.

7. Darreichungsform nach einem oder mehreren der vorangehenden Ansprüche, **dadurch gekennzeichnet, daß** die Partikel den/die Wirkstoff(e) in flüssiger Form enthalten, oder eine wirkstoffhaltige Lösung enthalten, welche mindestens einen festen Wirkstoff in gelöster Form in einem geeigneten Lösemittel enthält, vorzugsweise eine gesättigte Wirkstofflösung.

8. Darreichungsformen nach einem oder mehreren der vorangehenden Ansprüche, **dadurch gekennzeichnet, daß** zumindest ein Teil der wirkstoffbeladenen Partikel mit einem Überzug versehen sind, der die Diffusion des Wirkstoffs in die Umgebung zumindest vorübergehend behindert oder verlangsamt.

9. Darreichungsformen nach einem oder mehreren der vorangehenden Ansprüche, **dadurch gekennzeichnet, daß** sie Partikel enthalten, die mit verschiedenen Wirkstoffen beladen sind.

10. Darreichungsformen nach einem oder mehreren der vorangehenden Ansprüche, **dadurch gekennzeichnet, daß** unterschiedliche Partikel-Typen und -Größen eingesetzt werden.

11. Darreichungsformen nach einem oder mehreren der vorangehenden Ansprüche, **dadurch gekennzeichnet, daß** sie Partikel enthalten, die mit flüssigen Weichmachern und/oder Hautpenetrations-Enhancern beladen sind.

12. Darreichungsformen nach einem oder mehreren der vorangehenden Ansprüche, **dadurch gekennzeichnet, daß** sie Partikel enthalten, die wasserlöslich oder biodegradierbar sind.

13. Darreichungsformen nach einem oder mehreren der vorangehenden Ansprüche, **dadurch gekennzeichnet, daß** sie als transdermale therapeutische Systeme (TTS) formuliert sind, die eine wirkstoffundurchlässige Rückschicht und eine damit verbundene Trägermatrix aufweisen, wobei in die Trägermatrix eine Vielzahl von offen-porösen oder Kapillarräume enthaltenden Partikeln eingebettet sind, die als Wirkstoffreservoir dienen und mindestens einen Wirkstoff enthalten, vorzugsweise in flüssiger Form.

14. Transdermale therapeutische Systeme nach Anspruch 13, **dadurch gekennzeichnet, daß** die Trägermatrix haftklebende Eigenschaften aufweist und im Zustand vor der Applikation mit einer ablösbaren Schutzfolie bedeckt ist.

15. Transdermale therapeutische Systeme nach Anspruch 13 oder 14, **dadurch gekennzeichnet, daß** die Trägermatrix zwei- oder mehrschichtig ist, wobei in mindestens einer Schicht eine Vielzahl von offen-porösen oder Kapillarräume enthaltenden Partikeln eingebettet sind, die als Wirkstoffreservoir dienen und mindestens einen Wirkstoff enthalten, vorzugsweise in flüssiger Form.

16. Darreichungsformen nach einem oder mehreren der Ansprüche 1 bis 12, **dadurch gekennzeichnet, daß** sie als transmucosale Darreichungsformen formuliert sind und eine feste oder halbfeste Trägermatrix mit mucoadhäsiven Eigenschaften aufweisen, in die eine Vielzahl von offen-porösen oder Kapillarräume enthaltenden Partikeln eingebettet sind, die als Wirkstoffreservoir dienen und mindestens einen Wirkstoff enthalten, vorzugsweise in flüssiger Form.

17. Darreichungsformen nach einem oder mehreren der Ansprüche 1 bis 15, **dadurch gekennzeichnet, daß** sie als Wirkstoffpflaster zur topischen oder epikutanen Verabreichung von Wirkstoffen formuliert sind und eine feste oder halbfeste Trägermatrix aufweisen, in die eine Vielzahl von offenporösen oder Kapillarräume enthaltenden Partikeln eingebettet sind, die als Wirkstoffreservoir dienen und mindestens einen Wirkstoff enthalten, vorzugsweise in flüssiger Form.

18. Verfahren zur Herstellung von Arzneiformen zur Anwendung auf der Haut oder Schleimhaut, umfassend eine Trägermatrix und mindestens einen Wirkstoff, **gekennzeichnet durch** folgende Arbeitsschritte:
a) Bereitstellen des Trägermatrix-Materials in flüssiger oder halbfester Form, oder als Gel;
b) Bereitstellung eines flüssigen Wirkstoffes oder einer Wirkstofflösung, -dispersion, -suspension, -emulsion, oder einer flüssigen Wirkstoffzubereitung;
c) Mischen des flüssigen Wirkstoffs bzw. der Wirkstofflösung mit offen-porösen Partikeln aus natürlichen oder synthetischen Schwämmen oder aus verfestigten Schäumen, wodurch die Poren- oder Kapillarräume dieser Partikel mit Wirkstoffflüssigkeit oder -lösung gefüllt werden,
d) Abtrennen der Partikel von der überschüssigen Wirkstoffflüssigkeit bzw. -lösung;
e) Einbringen der wirkstoffhaltigen Partikel in das im ersten Schritt genannte Trägermaterial und Mischen;
f) falls erforderlich, Einstellung der gewünschten Konsistenz des Trägermaterials **durch** Lösemittelentzug, insbesondere **durch** Trocknen, oder Abkühlen.

19. Verfahren nach Anspruch 18, **dadurch gekennzeichnet, daß** die in Schritt (c) beschriebene Beladung der Partikel bei erhöhtem Druck oder unter Vakuumbedingungen erfolgt, vorzugsweise durch Kesseldruckimprägnierung.

20. verfahren nach Anspruch 18, **dadurch gekennzeichnet, daß** die in Schritt (c) beschriebene Beladung der Partikel auf die weise erfolgt, daß die Partikel enthaltende Wirkstoffflüssigkeit einem erhöhten Druck ausgesetzt und anschließend entspannt wird.

21. Verfahren nach Anspruch 18, **dadurch gekennzeichnet, daß** die in Schritt (c) beschriebene Beladung der Partikel auf die Weise erfolgt, daß die Partikel erhitzt werden und anschließend mit Wirkstoffflüssigkeit vermischt werden.

22. Verfahren zur Herstellung von Arzneiformen zur Anwen-, dung auf der Haut oder Schleimhaut, umfassend eine Trägermatrix und mindestens einen Wirkstoff, **gekennzeichnet durch** folgende Arbeitsschritte:
a) Bereitstellen des Trägermatrix-Materials in flüssiger oder halbfester Form, oder als Gel;
b) Mischen des flüssigen Wirkstoffs bzw. der Wirkstofflösung mit offen-porösen Partikeln aus natürlichen oder synthetischen Schwämmen oder aus verfestigten Schäumen, wodurch die Poren- oder Kapillarräume dieser Partikel mit Wirkstoffflüssigkeit oder -lösung gefüllt werden;
c) Bereitstellung eines flüssigen Wirkstoffes oder einer Wirkstofflösung, -dispersion, -suspension, -emulsion, oder einer flüssigen Wirkstoffzubereitung in einem Druckbehälter;
d) Auflösen eines inerten Gases in dem flüssigen Wirkstoff oder der Wirkstofflösung, unter erhöhtem Druck;
e) Entspannung der unter Druck stehenden Lösung aus Schritt (c), unter gleichzeitiger Beimischung eines festen, pulverförmigen Trägerstoffes, wobei ein flüssigkeitsbeladenes wirkstoffhaltigen Pulver entsteht;
f) Einbringen des wirkstoffhaltigen Pulvers aus Schritt (d) in das im ersten Schritt genannte Trägermaterial und Mischen;
g) falls erforderlich, Einstellung der gewünschten Konsistenz des Trägermaterials **durch** Lösemittelentzug, insbesondere **durch** Trocknen, oder Abkühlen.

## Claims

1. Administration forms for application on the skin or mucosa, comprising a carrier matrix with pressure sensitive adhesive or mucoadhesive properties and at least one active substance, said carrier matrix having a plurality of particles which serve as active substance reservoir and contain at least one active substance, **characterized in that** said particles
- are selected from the group comprising open-cell particles of natural sponges, open-cell particles of synthetic sponges, as well as open-cell particles of solidified foams.

2. Administration forms according to claim 1, **characterized in that** the average particle size of the particles is ≤ 2 mm, preferably ≤ 0.5 mm, more preferably ≤ 200 µm.

3. Administration forms according to claim 1 or 2 **characterized in that** the particles having pores or capillary spaces are finely pored, with an average pore or capillary diameter of ≤ 0.1 mm, preferably ≤ 20 µm, especially ≤ 1 µm.

4. Administration forms according to any one of the preceding claims, **characterized in that** the portion of the particles, relative to the carrier matrix, amounts to 0.1 to 95%-wt, preferably 5 to 60%-wt.

5. Administration form according to any one of the preceding claims, **characterized in that** the particles contain swellable, liquid-absorbing polymers, preferably superabsorbing polymers, as carrier material, with water-swellable polymers, especially polyvinyl alcohol having a high degree of hydrolysis, and high-molecular hydroxypropylmethyl cellulose being particularly preferred.

6. Administration forms according to any one of the preceding claims, **characterized in that** said pulverulent carrier material is selected from the group comprising
- starch derivatives, especially maize starch, potato starch, wheat starch,
- silicic acid and silicon dioxide celluloses, especially microcrystalline celluloses, cellulose derivatives such as carboxymethyl cellulose, cellulose fibres,
- activated charcoal, zeolite and silicic acid.

7. Administration form according to one or more of the preceding claims, **characterized in that** the particles contain the active substance(s) in liquid form or contain an active substance-containing solution which contains at least one solid active substance in dissolved form in a suitable solvent, preferably a saturated active substance solution.

8. Administration forms according to one or more of the preceding claims, **characterized in that** at least a part of the active substance-loaded particles is provided with a coating which inhibits or slows down the diffusion of the active substance to its environment at least temporarily.

9. Administration forms according to one or more of the preceding claims, **characterized in that** they contain particles loaded with different active substances.

10. Administration forms according to one or more of the preceding claims **characterized in that** different particle types and particle sizes are used.

11. Administration forms according to one or more of the preceding claims, **characterized in that** they contain particles loaded with liquid plasticizers and/or skin-penetration enhancers.

12. Administration forms according to one or more of the preceding claims, **characterized in that** they contain particles which are water-soluble or biodegradable.

13. Administration forms according to one or more of the preceding claims, **characterized in that** they are formulated as transdermal therapeutic systems (TTS) which have an active substance-impermeable backing layer and a carrier matrix connected thereto, with a plurality of particles being embedded in the carrier matrix which have open pores or contain capillary spaces and which serve as active substance reservoir and contain at least one active substance, preferably in liquid form.

14. Transdermal therapeutic systems according to claim 13 **characterized in that** the carrier matrix has pressure-sensitive adhesive properties and, in the state prior to application, is covered with a detachable protective film.

15. Transdermal therapeutic systems according to claim 13 or 14, **characterized in that** the carrier matrix is a bilayer or mono-layer matrix, with a plurality of particles being embedded in at least one layer thereof which have open pores or contain capillary spaces and which serve as active substance reservoir and contain at least one active substance, preferably in liquid form.

16. Administration forms according to one or more of claims 1 to 12, **characterized in that** they are formulated as transmucosal administration forms and have a solid or semi-solid carrier matrix, in which matrix there is embedded a plurality of particles which have open pores or contain capillary spaces and which serve as active substance reservoir and contain at least one active substance, preferably in liquid form.

17. Administration forms according to one or more of claims 1 to 15, **characterized in that** they are formulated as active substance plasters for topical or epicutaneous administration of active substances and comprise a solid or semi-solid carrier matrix, in which matrix there is embedded a plurality of particles which have open pores or contain capillary spaces and which serve as active substance reservoir and contain at least one active substance, preferably in liquid form.

18. Process for the production of forms of medicaments for application on the skin or mucosa, comprising a carrier matrix and at least one active substance, **characterized by** the following steps:
a) providing the carrier matrix material in liquid or semi-solid form, or as a gel;
b) providing a liquid active substance or an active substance solution, dispersion, suspension or emulsion, or a liquid active substance preparation;
c) mixing the liquid active substance, respectively the active substance solution, with open-cell particles made of natural or synthetic sponges or of solidified foams, thereby filling the pores or capillary spaces of these particles with active substance liquid or active substance solution;
d) separating the particles from the excess active substance fluid or solution;
e) introducing the active substance-containing particles into the carrier material mentioned in the first step, and mixing;
f) if necessary, adjusting the desired consistency of the carrier material by solvent withdrawal, especially by drying, or cooling.

19. The process according to claim 18, **characterized in that** the loading of the particles described in step (c) is carried out at increased pressure or under vacuum conditions, preferably by pressure impregnation in a pressurized chamber.

20. The process according to claim 18, **characterized in that** the loading of the particles described in step (c) is performed in such a manner that the particle-containing active substance fluid is subjected to increased pressure and the pressure is subsequently relieved.

21. The process according to claim 18, **characterized in that** the loading of the particles described in step (c) is performed in such a manner that the particles are heated and subsequently mixed with active substance liquid.

22. Process for the production of forms of medicaments for application on the skin or mucosa, comprising a carrier matrix and at least one active substance, **characterized by** the following steps:
a) providing the carrier matrix material in liquid or semi-solid form, or as a gel;
b) mixing the liquid active substance, respectively the active substance solution, with open-cell particles made of natural or synthetic sponges or of solidified foams, thereby filling the pores or capillary spaces of these particles with active substance liquid or active substance solution;
c) providing a liquid active substance or an active substance solution, dispersion, suspension or emulsion, or a liquid active substance preparation, in a pressurized vessel;
d) dissolving an inert gas in the liquid active substance or active substance solution, under increased pressure;
e) relieving the pressure on the pressurized solution from step (c), while simultaneously admixing a solid, pulverulent carrier substance, whereby a liquid-loaded active substance-containing powder is formed;
f) introducing the active substance-containing powder from step (d) into the carrier material mentioned in the first step, and mixing;
g) if necessary, adjusting the desired consistency of the carrier material by solvent withdrawal, especially by drying, or cooling.

## Revendications

1. Formes posologiques à appliquer sur la peau ou sur une muqueuse, comprenant une matrice de support possédant des propriétés autoadhésives ou des propriétés mucoadhésives, et au moins une substance active, la matrice de support présentant une multitude de particules qui font office de réservoir de substances actives et qui contiennent au moins une substance active, **caractérisées en ce que** les particules mentionnées sont choisies parmi le groupe qui comprend des particules à pores ouverts constituées par les éponges naturelles, des particules à pores ouverts constituées par des éponges synthétiques et des particules à pores ouverts constituées par des mousses solidifiées.

2. Formes posologiques selon la revendication 1, **caractérisées en ce que** la granulométrie moyenne des particules est ≤ 2 mm, de préférence ≤ 0,5 mm, de manière plus préférée ≤ 200 µm.

3. Formes posologiques selon la revendication 1 ou 2, **caractérisées en ce que** les particules poreuses ou présentant des espaces capillaires sont des particules finement poreuses possédant un diamètre moyen des pores, respectivement des capillaires ≤ 0,1 mm, de préférence ≤ 20 µm, en particulier ≤ 1 µm.

4. Formes posologiques selon une ou plusieurs des revendications précédentes, **caractérisées en ce que** la fraction des particules, rapportée à la matrice de support, s'élève de 0,1 à 95 % en poids, de préférence de 5 à 60 % en poids.

5. Forme posologique selon une ou plusieurs des revendications précédentes, **caractérisée en ce que** les particules contiennent, à titre de matière de support, des polymères aptes à gonfler absorbant les liquides, de préférence des polymères superabsorbants, des polymères aptes à gonfler dans l'eau, en particulier de l'alcool polyvinylique possédant un degré d'hydrolyse élevé et de l'hydroxypropylméthylcellulose à poids moléculaire élevé étant particulièrement préférés.

6. Formes posologiques selon une ou plusieurs des revendications précédentes, **caractérisées en ce que** la matière de support pulvérulente mentionnée est choisie parmi le groupe qui comprend :
- des types d'amidon en particulier l'amidon de maïs, l'amidon de pomme de terre, l'amidon de froment ;
- de l'acide silicique et du dioxyde de silicium, des celluloses, en particulier des celluloses microcristallines, des dérivés de la cellulose tels que la carboxyméthylcellulose, des fibres cellulosiques ;
- du charbon actif, des zéolithes et de l'acide silicique.

7. Forme posologique selon une ou plusieurs des revendications précédentes, **caractérisée en ce que** les particules contiennent la/les substances actives sous forme liquide, ou contiennent une solution contenant une ou plusieurs substances actives, qui contient au moins une substance active solide sous forme dissoute dans un solvant approprié, de préférence une solution saturée de substance(s) active(s).

8. Formes posologiques selon une ou plusieurs des revendications précédentes, **caractérisées en ce que** au moins une partie des particules chargées de substance(s) active(s) est munie d'un enrobage qui ralentit ou qui entrave au moins de manière temporaire la diffusion de la ou des substances actives dans l'environnement.

9. Formes posologiques selon une ou plusieurs des revendications précédentes, **caractérisées en ce qu'**elles contiennent des particules qui sont chargées avec des substances actives différentes.

10. Formes posologiques selon une ou plusieurs des revendications précédentes, **caractérisées en ce que** différents types de particules et différentes dimensions de particules sont mis en oeuvre.

11. Formes posologiques selon une ou plusieurs des revendications précédentes, **caractérisées en ce qu'**elles contiennent des particules qui sont chargées avec des plastifiants liquides et/ou avec des amplificateurs de la pénétration dans la peau.

12. Formes posologiques selon une ou plusieurs des revendications précédentes, **caractérisées en ce qu'**elles contiennent des particules qui sont solubles dans l'eau ou qui sont biodégradables.

13. Formes posologiques selon une ou plusieurs des revendications précédentes, **caractérisées en ce qu'**elles sont formulées pour obtenir des systèmes thérapeutiques transdermiques (TTS) qui présentent une couche dorsalé imperméable à la substance active/aux substances actives et une matrice de support qui y est liée, une multitude de particules à pores ouverts ou contenant des espaces capillaires étant incorporées dans la matrice de support, lesdites particules faisant office de réservoir de substance(s) active(s) et contenant au moins une substance active, de préférence sous forme liquide.

14. Systèmes thérapeutiques transdermiques selon la revendication 13, **caractérisés en ce que** la matrice de support présente des propriétés autoadhésives et est recouverte, à l'état précédant l'application, d'une feuille de protection détachable.

15. Systèmes thérapeutiques transdermiques selon la revendication 13 ou 14, **caractérisés en ce que** la matrice de support est une matrice bicouche ou multicouche, une multitude de particules à pores ouverts ou contenant des espaces capillaires étant incorporées dans au moins une couche, lesdites particules faisant office de réservoir de substance(s) active(s) et contenant au moins une substance active, de préférence sous forme liquide.

16. Formes posologiques selon une ou plusieurs des revendications 1 à 12, **caractérisées en ce qu'**elles sont formulées pour obtenir des formes posologiques transmuqueuses et présentent une matrice de support solide ou semi-solide possédant des propriétés mucoadhésives, une multitude de particules à pores ouverts ou contenant des espaces capillaires étant incorporées dans ladite matrice de support, lesdites particules faisant office de réservoir de substance(s) active(s) et contenant au moins une substance active, de préférence sous forme liquide.

17. Formes posologiques selon une ou plusieurs des revendications 1 à 15, **caractérisées en ce qu'**elles sont formulées pour obtenir des emplâtres contenant une ou plusieurs substances actives pour l'administration locale ou épicutanée de substance(s) active(s) et présentent une matrice de support solide ou semi-solide, une multitude de particules à pores ouverts ou contenant des espaces capillaires étant incorporées dans ladite matrice de support, lesdites particules faisant office de réservoir de substance(s) active(s) et contenant au moins une substance active, de préférence sous forme liquide.

18. Procédé pour la préparation de formes médicamenteuses à appliquer sur la peau ou sur une muqueuse, comprenant une matrice de support et au moins une substance active, **caractérisé par** les étapes opératoires suivantes :
a) fournir la matière de la matrice de support sous forme liquide ou sous forme semi-solide ou encore sous forme d'un gel;
b) fournir une substance active liquide ou bien une solution, une dispersion, une suspension, une émulsion contenant une ou plusieurs substances actives, ou encore une préparation liquide contenant une ou plusieurs substances actives ;
c) mélanger la substance active liquide respectivement la solution contenant une ou plusieurs substances actives avec des particules à pores ouverts constituées par des éponges naturelles ou synthétiques ou par des mousses solidifiées, les espaces poreux ou les espaces capillaires de ces particules étant remplis avec un liquide ou avec une solution contenant une ou plusieurs substances actives ;
d) séparer les particules du liquide, respectivement de la solution en excès contenant une ou plusieurs substances actives ;
e) incorporer les particules contenant une ou plusieurs substances actives dans la matière de support mentionnée dans la première étape, et mélanger ;
f) en cas de nécessité, régler la consistance désirée de la matière de support par élimination du solvant, en particulier par séchage ou par refroidissement.

19. Procédé selon la revendication 18, **caractérisé en ce que** le chargement des particules, décrit à l'étape (c), a lieu sous pression élevée ou dans des conditions de vide, de préférence via une imprégnation en autoclave sous pression.

20. Procédé selon la revendication 18, **caractérisé en ce que** le chargement des particules, décrit à l'étape (c), a lieu de telle sorte que le liquide contenant une ou plusieurs substances actives et qui contient des particules est exposé à une pression élevée et est ensuite soumis à une détente.

21. Procédé selon la revendication 18, **caractérisé en ce que** le chargement des particules, décrit à l'étape (c), a lieu de telle sorte que les particules sont chauffées et ensuite sont mélangées avec un liquide contenant une ou plusieurs substances actives.

22. Procédé pour la préparation de formes médicamenteuses à appliquer sur la peau ou sur une muqueuse, comprenant une matrice de support et au moins une substance active, **caractérisé par** les étapes opératoires suivantes :
a) fournir la matière de la matrice de support sous forme liquide ou sous forme semi-solide ou encore sous forme d'un gel ;
b) mélanger la substance active liquide respectivement la solution contenant une ou plusieurs substances actives avec des particules à pores ouverts constituées par des éponges naturelles ou synthétiques ou par des mousses solidifiées, les espaces poreux ou les espaces capillaires de ces particules étant remplis avec un liquide ou avec une solution contenant une ou plusieurs substances actives ;
c) fournir une substance active liquide ou bien une solution, une dispersion, une suspension, une émulsion contenant une ou plusieurs substances actives, ou encore une préparation liquide contenant une ou plusieurs substances actives dans un récipient mis sous pression;
d) solubiliser un gaz inerte dans la substance active liquide ou dans la solution contenant une ou plusieurs substances actives, sous pression élevée ;
e) détendre la solution mise sous pression à l'étape c), tout en introduisant simultanément par mélange une substance de support solide, pulvérulente, pour obtenir une poudre contenant une ou plusieurs substances actives, chargée d'un liquide ;
f) incorporer la poudre de l'étape d) contenant une ou plusieurs substances actives dans la matière de support mentionnée à la première étape et mélanger ;
g) en cas de nécessité, régler la consistance désirée de la matière de support par élimination du solvant, en particulier par séchage ou par refroidissement.
